(19) Europäisches Patentamt
European Patent Office
Office européen des brevets

(11) Veröffentlichungsnummer: **0 653 677 A1**

(12) **EUROPÄISCHE PATENTANMELDUNG**

(21) Anmeldenummer: **94117334.6**

(51) Int. Cl.[6]: **G03C 7/34**

(22) Anmeldetag: **03.11.94**

(30) Priorität: **16.11.93 DE 4339121**

(43) Veröffentlichungstag der Anmeldung:
**17.05.95 Patentblatt 95/20**

(84) Benannte Vertragsstaaten:
**DE FR GB**

(71) Anmelder: **Agfa-Gevaert AG**
**Kaiser-Wilhelm-Allee**
**D-51373 Leverkusen (DE)**

(72) Erfinder: **Wolff, Erich, Dr.**
**Balkhauser Weg 6**
**D-42659 Solingen (DE)**
Erfinder: **Wiesen, Heinz**
**Kieselweg 16**
**D-53881 Euskirchen (DE)**
Erfinder: **Langen, Hans, Dr.**
**Weidengarten 16**
**D-53129 Bonn (DE)**

(54) **Fotografisches Farbkupplerhaltiges Aufzeichungsmaterial.**

(57) Farbfotografisches Aufzeichnungsmaterial mit mindestens einer für den roten Spektralbereich sensibilisierten Silberhalogenidemulsionsschicht und einem Cyankuppler der nachstehenden Formel I. Die Cyankuppler der Formel I zeichnen sich durch eine steile Gradation, eine hohe maximale Farbdichte und eine hohe Farbausbeute aus.

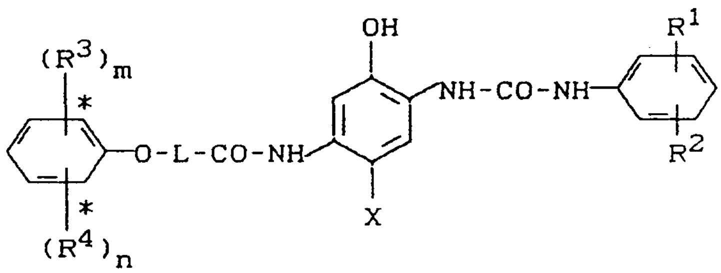

(I)

In Formel I bedeuten

- X — H oder eine von Wasserstoff verschiedene, bei Farbkupplung freisetzbare Gruppe;
- L — ein zweibindiges aliphatisches Bindeglied, insbesondere Alkylen oder Alkyliden mit 1 - 17 C-Atomen;
- $R^1$ — einen elektronenanziehenden Substituenten, z.B. -F, $-CF_3$, -CN, Carbamoyl, Alkoxycarbonyl, Alkylsulfonyl, Sulfamoyl, $-SO_2F$;
- $R^2$ — H, Cl, Alkyl, Alkoxy, Alkylthio, Acylamino oder einen Rest wie $R^1$;
- $R^3$ — Cycloalkyl;
- $R^4$ — Halogen oder Alkoxy mit 1 bis 12 C-Atomen, wobei fall $R^4$ Alkoxy bedeutet, beide ortho-Positionen (*) substituiert sind;
- m — 1 oder 2;
- n — 1, 2 oder 3.

EP 0 653 677 A1

Die Erfindung betrifft ein farbfotografisches Aufzeichnungsmaterial, das einen einemulgierten phenolischen Cyankuppler mit Phenylureidostruktur und einer speziellen Ballastgruppe enthält. Das Aufzeichnungsmaterial weist in der rotempfindlichen Schicht eine deutlich verbesserte Kupplungsaktivität auf.

Es ist bekannt, farbige fotografische Bilder durch chromogene Entwicklung herzustellen, d. h. dadurch, daß man bildmäßig belichtete Silberhalogenidemulsionsschichten in Gegenwart geeigneter Farbkuppler mittels geeigneter farbbildender Entwicklersubstanzen - sogenannter Farbentwickler - entwickelt, wobei das in Übereinstimmung mit dem Silberbild entstehende Oxidationsprodukt der Entwicklersubstanzen mit dem Farbkuppler unter Bildung eines Farbstoffbildes reagiert. Als Farbkuppler werden gewöhnlich aromatische, primäre Aminogruppen enthaltende Verbindungen, insbesondere solche vom p-Phenylendiamintyp, verwendet.

Für die Herstellung des blaugrünen Teilbildes werden üblicherweise naphtholische oder phenolische Cyankuppler verwendet In farbfotografischen Aufzeichnungsmaterialien gab man bisher den ersteren den Vorzug wegen der günstigeren Absorption (bei ca. 700 nm) der aus ihnen bei der chromogenen Entwicklung erzeugten Bildfarbstoffe. Die phenolischen Cyankuppler liefern demgegenüber in der Regel Farbstoffe mit einem Absorptionsmaximum bei kürzeren Wellenlängen.

Obwohl die naphtholischen Cyankuppler besonders bei Anwendung in Farbnegativfilmen in spektraler Hinsicht ideal sind, besteht ein gravierender Nachteil in den unzureichenden Stabilitätseigenschaften der Farbstoffe, insbesondere in der mangelhaften Stabilität gegenüber Feuchtigkeit und Wärme. In dieser Hinsicht sind den naphtholischen Cyankupplern die phenolischen vorzuziehen.

In EP-A-0 028 099 und EP-A-0 067 689 sind phenolische Cyankuppler beschrieben, die in der 2-Stellung des Phenolringes eine im Benzolring substituierte Phenylureidogruppe enthalten. Diese Kuppler liefern bei Farbentwicklung Farbstoffe mit guter Stabilität gegenüber der Einwirkung von Wärme, Feuchte und Licht und einem vergleichsweise langwelligem Absorptionsmaximum, die sich darüber hinaus durch eine vergleichsweise geringe Halbbandbreite (hbw) auszeichnen. Sie sind aber bezüglich einer hohen Kupplungsaktivität weiter verbesserungsbedürftig. Eine hohe Reaktivität im obigen Sinne führt zu einer steilen Gradation, einer hohen maximalen Dichte und in einigen Fällen zu einer schnellen Kupplungskinetik. Hohe maximale Dichte kann einen geringen Kupplerauftrag zur Folge haben, der seinerseits eine erhebliche Kosteneinsparung bedeutet.

Bei der heutigen Technologie mit emulgierten Farbkupplern tragen die einzelnen Kupplermoleküle sogenannte Ballastreste, die eine Diffusion des Kupplers in benachbarte Schichtbereiche verhindern. Diese Ballastreste beeinflussen darüber hinaus die Löslichkeit der Farbkuppler in hochsiedenden organischen Lösungsmitteln sogenannten Ölbildnern und bewirken außerdem eine gute Stabilität der damit hergestellten Kuppleremulgate bei Kühllagerung oder während des Herstellungsprozesses.

Die Anforderungen in dieser Hinsicht sind in den letzten Jahren drastisch gestiegen. Es wurden neue Cyankuppler der nachfolgend beschriebenen Grundstruktur gefunden, die eine ausgezeichnete Kupplungsaktivität im Vergleich zu herkömmlichen Kupplern haben.

Gegenstand der Erfindung ist ein farbfotografisches Aufzeichnungsmaterial mit mindestens einer für den roten Spektralbereich sensibilisierten Silberhalogenidemulsionsschicht, der ein Cyankuppler vom Typ des 2-Phenylureidophenols zugeordnet ist, dadurch gekennzeichnet, daß der Cyankuppler der Formel I entspricht.

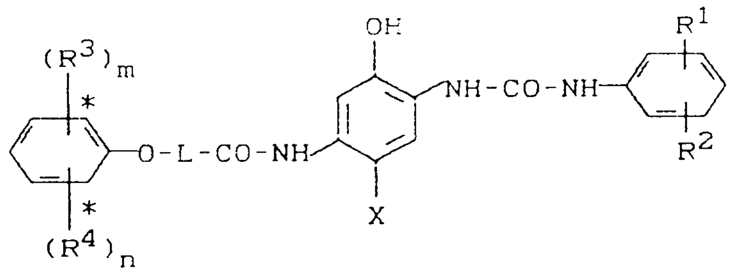

(I)

worin

- X H oder eine von Wasserstoff verschiedene, bei Farbkupplung freisetzbare Gruppe;
- L ein zweibindiges aliphatisches Bindeglied, insbesondere Alkylen oder Alkyliden mit 1 - 17 C-Atomen;
- $R^1$ einen elektronenanziehenden Substituenten, z.B. -F, $-CF_3$, -CN, Carbamoyl, Alkoxycarbonyl, Alkylsulfonyl, Sulfamoyl, $SO_2F$;

$R^2$ H, Cl, Alkyl, Alkoxy, Alkylthio, Acylamino oder einen Rest wie $R^1$;
$R^3$ Cycloalkyl, z.B. Cyclopentyl oder Cyclohexyl;
$R^4$ Halogen oder Alkoxy mit 1 bis 12 C-Atomen, wobei falls $R^4$ Alkoxy bedeutet, beide ortho-Positionen (*) substituiert sind;
m 1 oder 2;
n 1, 2 oder 3.

Beispiele für Cyankuppler der Formel I sind nachfolgend angegeben:

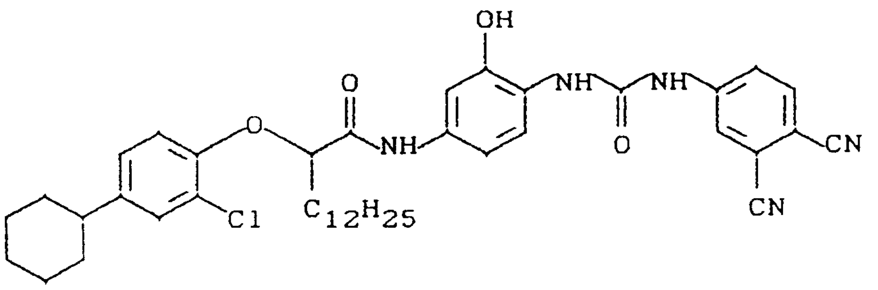

1

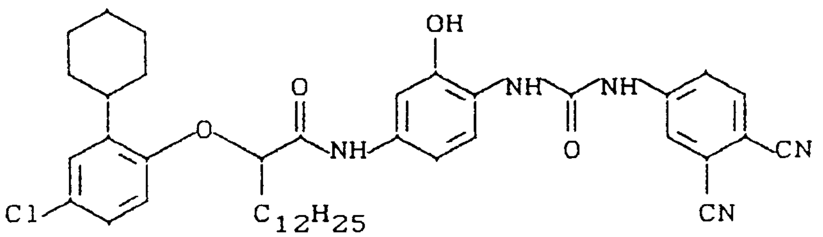

2

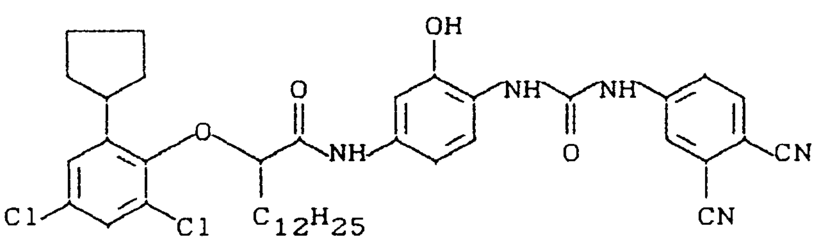

3

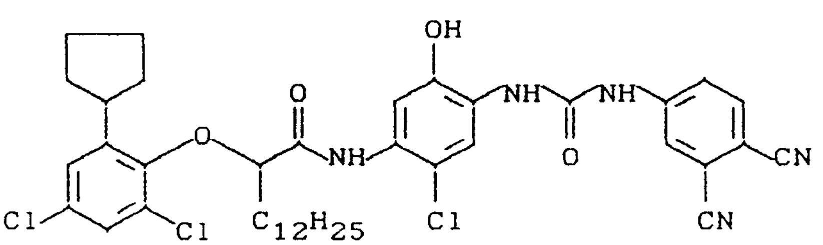

4

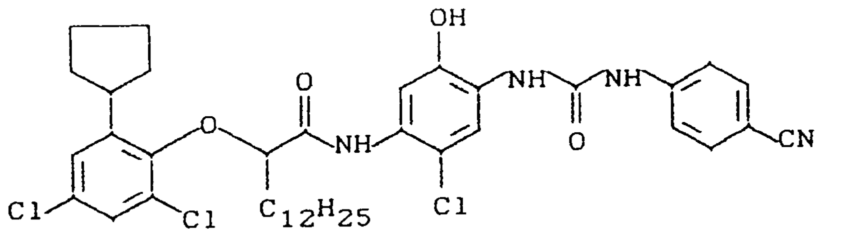

5

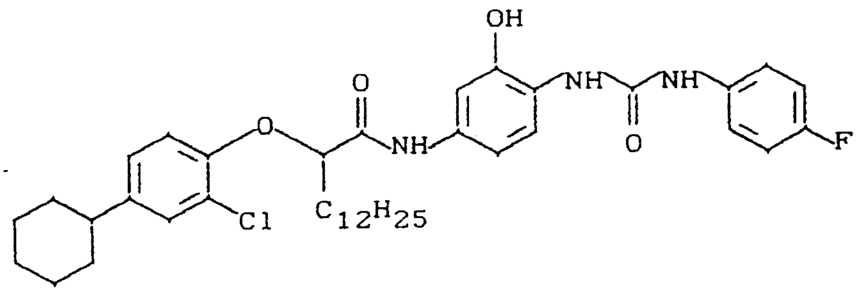

.6

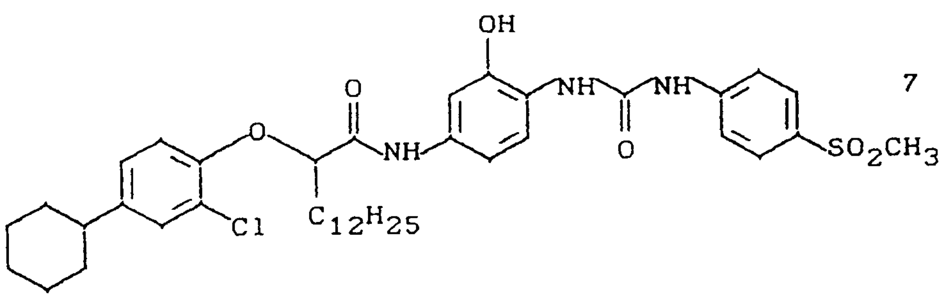

7

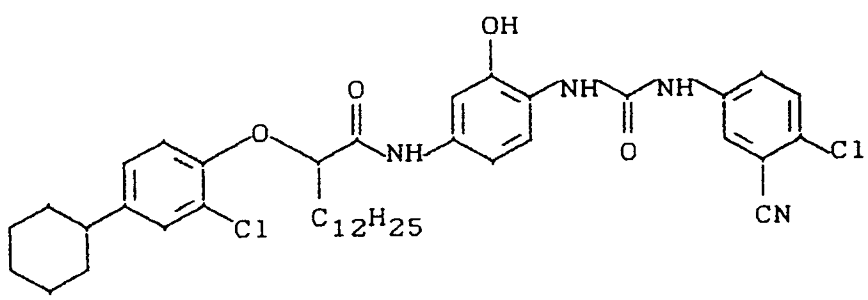

8

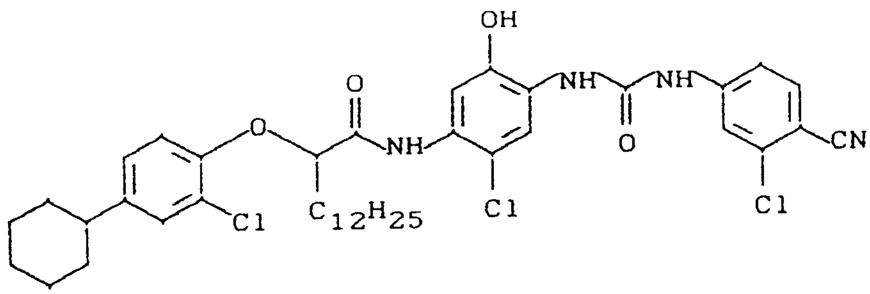

9

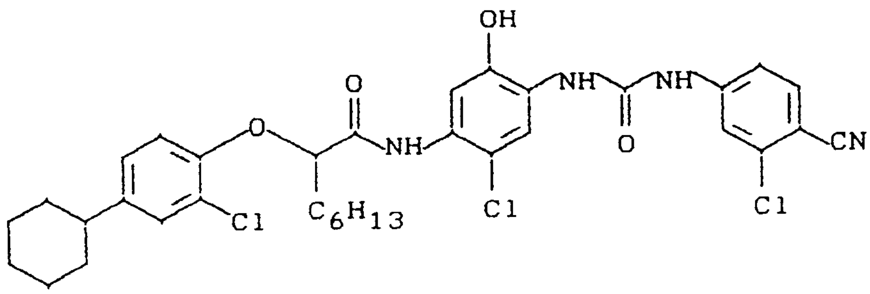

10

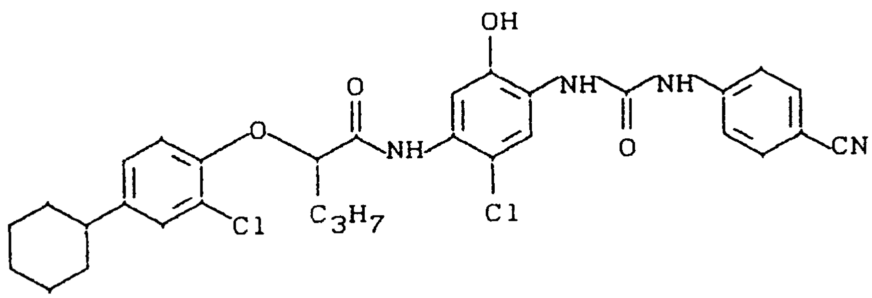

11

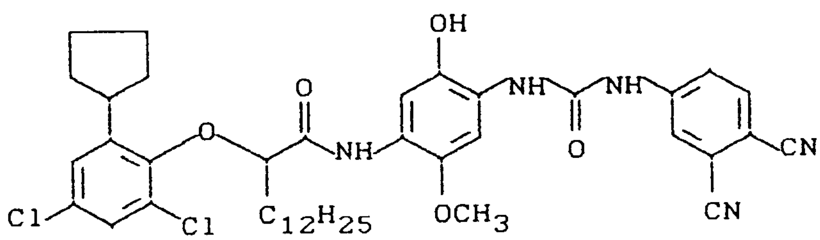

12

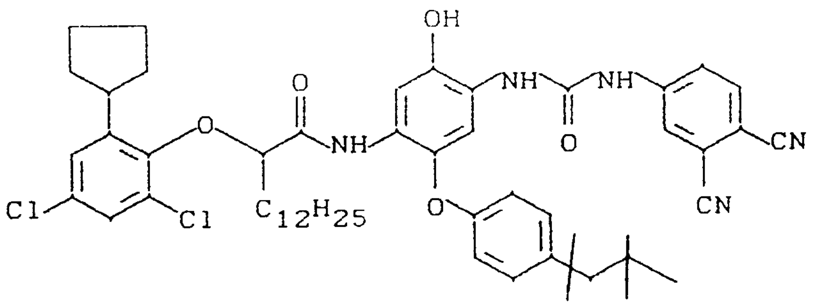

13

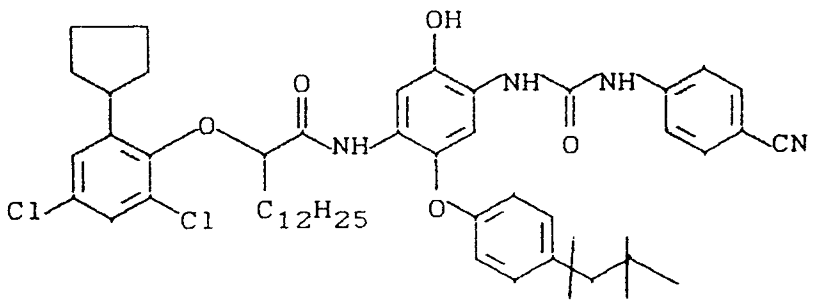

14

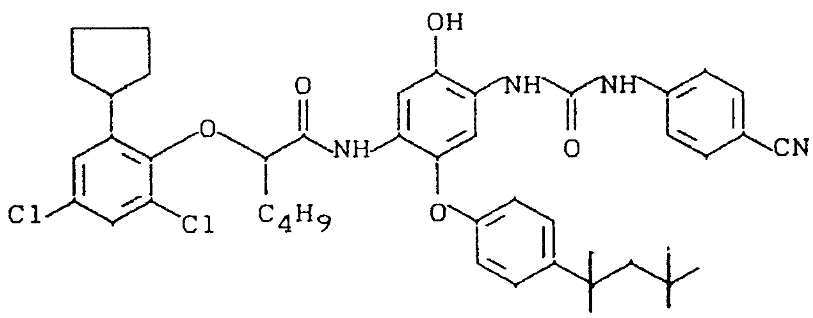

15

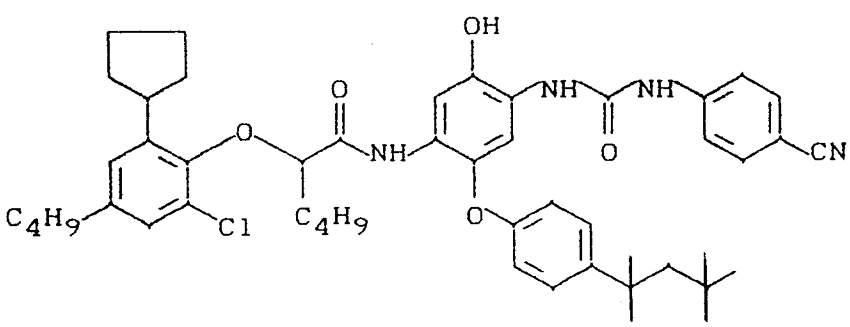

16

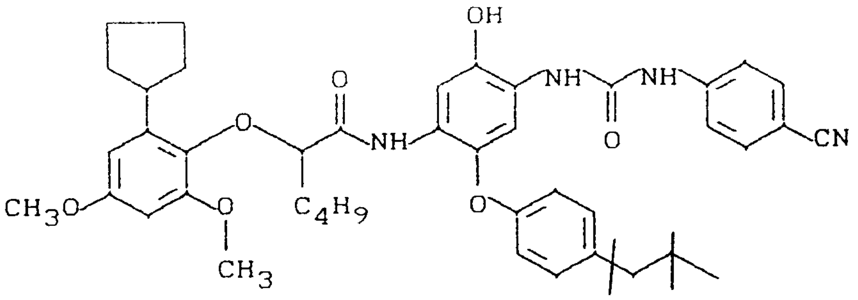

17

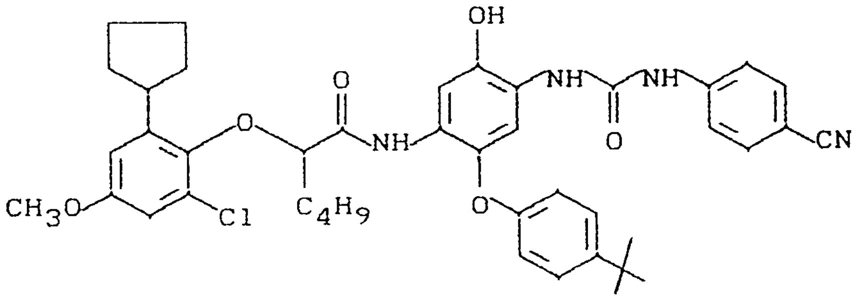

18

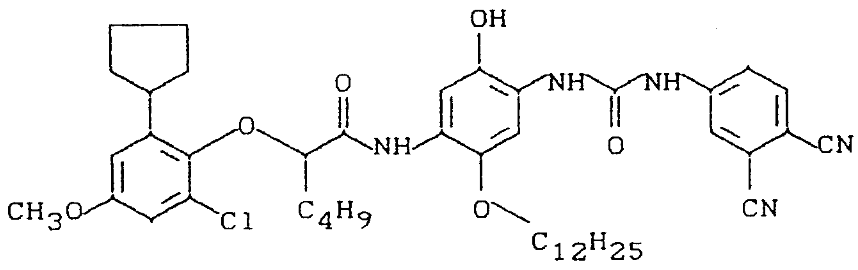

19

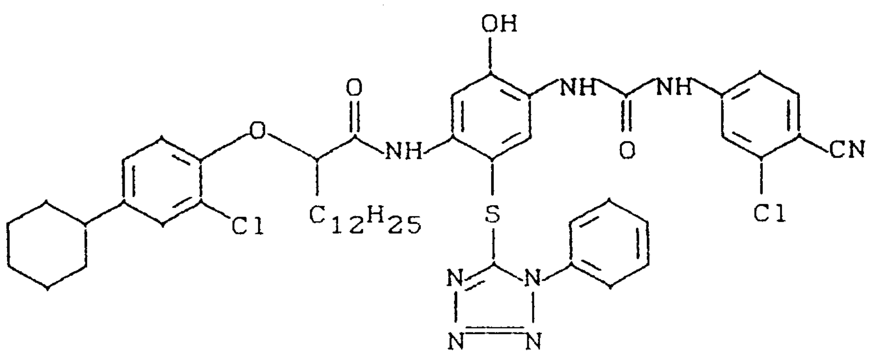

20

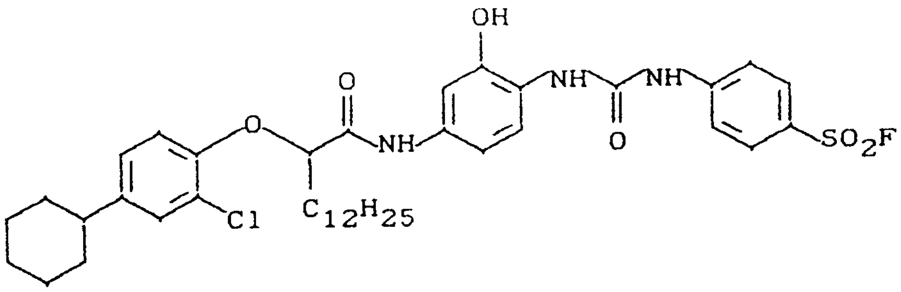

21

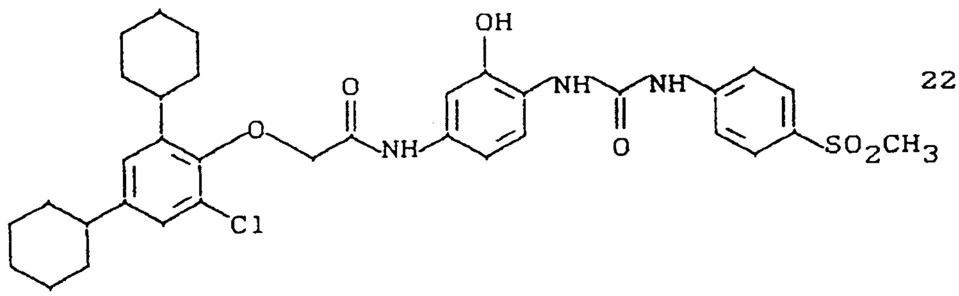

22

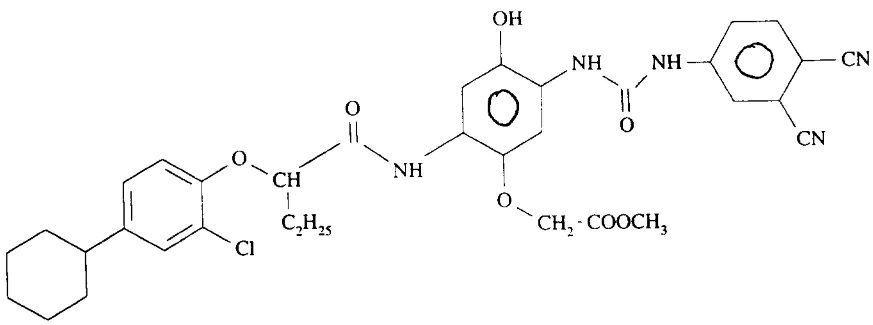

23

Syntheseschema:

Die erfindungsgemäßen Verbindungen können beispielsweise auf dem skizzierten Wege synthetisiert werden.

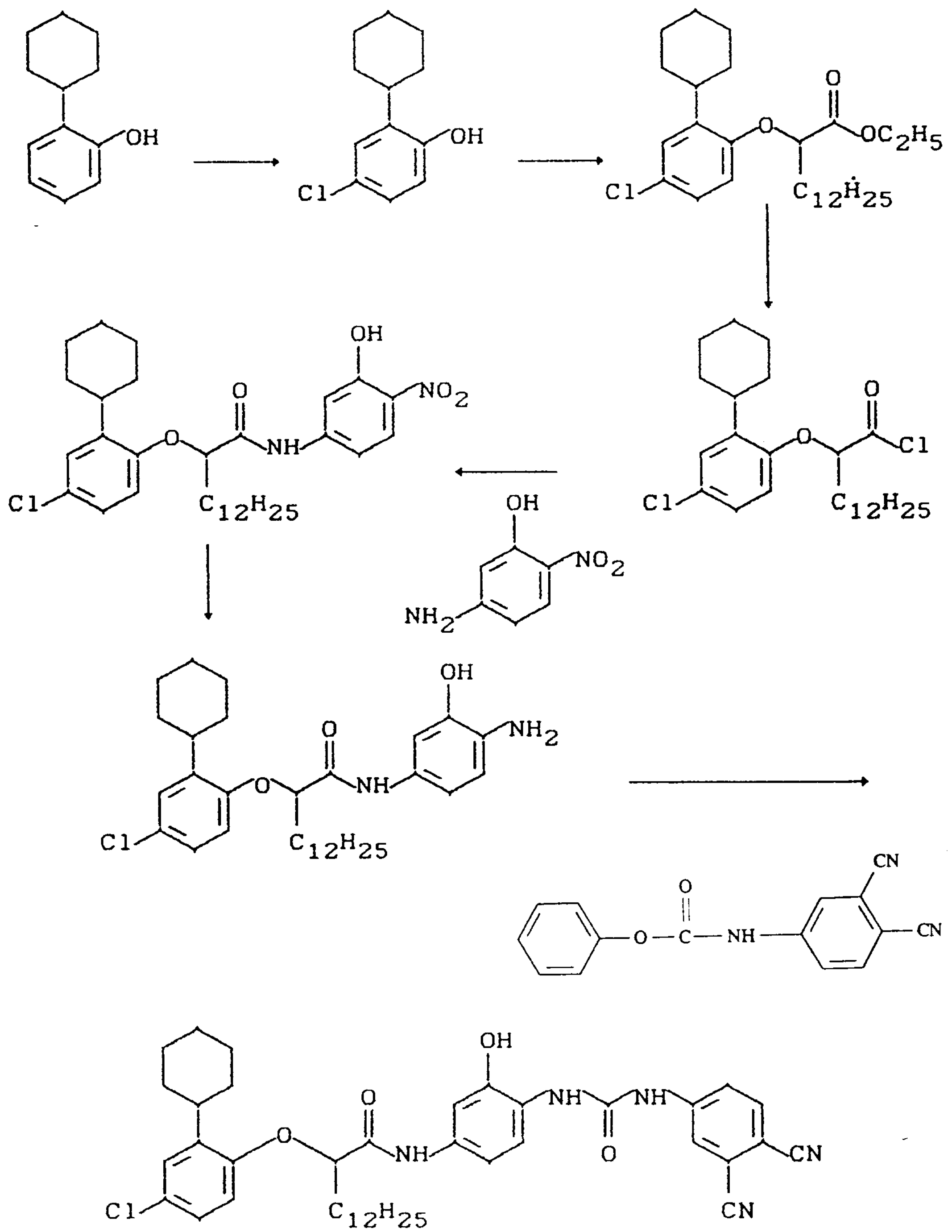

Synthesevorschrift für Kuppler Nr. 1

21,05 g 2-Chlor-4-cyclohexylphenol in 300 ml wasserfreiem Xylol vorlegen und auf 120 °C erhitzen. 19 g 30 %ige Natriummethylatlösung zutropfen und dabei das eingebrachte und entstehende Methanol bis zur Kopftemperatur von 138 °C abdestillieren.

33,5 g 2-Brommyristinsäureester in 30 min zudosieren und bei schwachem Sieden 4 h rühren. Auf 110 °C abkühlen, 12 ml kohzentrierter Natronlauge und 12 ml Wasser einlaufen lassen und zur Hydrolyse des Esters 2 h bei Siedetemperatur rühren. Auf Raumtemperatur abkühlen und die organische Phase abtrennen.

Nach Verrühren mit 20 %iger Salzsäure und Waschen mit Wasser wird das Xylol im Vakuum nahezu vollständig entfernt.

Dem zurückbleibenden Öl bei 50-60 °C Innentemperatur 75 ml Thionylchlorid zudosieren und bei 100 °C Badtemperatur 3 h bis zum Ende der Gasentwicklung rühren.

Überschüssiges Thionylchlorid durch Destillation entfernen.

25,4 g 2-Phthalimido-5-amino-phenol werden in 400 ml Acetonitril suspendiert und unter Rühren mit 50 g des obigen Säurechlorids 2,5 Stunden zum Sieden erhitzt. Nach ungefähr 1 Stunde entsteht eine klare Lösung. Anschließend wird auf 70 °C abgekühlt und heiß filtriert. Unter Rühren wird langsam auf 10 °C abgekühlt und anschließend abgesaugt. Es wird mit 75 ml kaltem Acetonitril gewaschen und danach mit 300 ml Methanol aufgekocht. Es wird bei Raumtemperatur abgesaugt und mit 50 ml Methanol gewaschen und getrocknet.

100,8 g des so hergestellten Produktes werden unter Rühren in 420 ml Toluol eingetragen. Bei Raumtemperatur werden 17,55 ml Hydrazinhydrat zugefügt. Es wird 1 Stunde nachgerührt und das ausgefallene Phthalhydrazid abgesaugt. Die Toluolphase wird mit 300 ml 5 %iger Kochsalzlösung verrührt und mit 13 ml 2n Salzsäure auf genau pH = 3 eingestellt. Zur besseren Phasentrennung werden 300 ml Aceton zugefügt. Die organische Phase wird abgetrennt und im Wasserstrahlvakuum um 220 ml eingeengt. Anschließend werden 39,6 g 3,4-Dicyanophenylcarbamidsäurephenylester unter Stickstoff zugefügt und auf 80 °C Innentemperatur erwärmt. Bei dieser Temperatur werden innerhalb von 30 Minuten 2 ml Triethylamin zugetropft und 2 Stunden bei 80 bis 85 °C nachgerührt. Es wird auf 40 °C abgekühlt und 1 ml Eisessig zugefügt. Im Vakuum werden 170 ml Toluol abdestilliert. Zum Rückstand werden 530 ml Methanol gegeben und auf 10 °C abgekühlt. Anschließend wird abgesaugt und mit 100 ml kaltem Methanol gewaschen. Ausbeute nach Umkristallisation aus Essigester/Methanol: 71 g.

Bei der Herstellung des erfindungsgemäßen farbfotografischen Aufzeichnungsmaterials kann der diffusionsfeste Cyankuppler der vorliegenden Erfindung in bekannter Weise in die Gießlösung der Silberhalogenidemulsionsschichten oder anderer Kolloidschichten eingearbeitet werden. Beispielsweise können die bevorzugt öllöslichen oder hydrophoben Kuppler aus einer Lösung in einem geeigneten Kupplerlösungsmittel (Ölbildner) gegebenenfalls in Anwesenheit eines Netz- oder Dispergiermittels zu einer hydrophilen Kolloidlösung zugefügt werden. Die hydrophile Gießlösung kann selbstverständlich neben dem Bindemittel andere übliche Zusätze enthalten. Die Kupplerlösung braucht nicht direkt in die Gießlösung für die Silberhalogenidemulsionsschicht oder eine andere wasserdurchlässige Schicht dispergiert zu werden; sie kann vielmehr auch vorteilhaft zuerst in einer wäßrigen nichtlichtempfindlichen Lösung eines hydrophilen Kolloids dispergiert werden, worauf das erhaltene Gemisch gegebenenfalls nach Entfernung der verwendeten niedrig siedenden organischen Lösungsmittel mit der Gießlösung für die lichtempfindliche Silberhalogenidemulsionsschicht oder einer anderen wasserdurchlässigen Schicht vor dem Auftragen vermischt wird.

Das als lichtempfindlicher Bestandteil in dem fotografischen Material befindliche Silberhalogenid kann als Halogenid Chlorid, Bromid oder Iodid bzw. Mischungen davon enthalten. Beispielsweise kann der Halogenidanteil wenigstens einer Schicht zu 0 bis 15 mol-% aus Iodid, zu 0 bis 100 mol-% aus Chlorid und zu 0 bis 100 mol-% aus Bromid bestehen. Im Falle von Farbnegativ- und Farbumkehrfilmen werden üblicherweise Silberbromidiodidemulsionen, im Falle von Farbnegativ- und Farbumkehrpapier üblicherweise Silberchloridbromidemulsionen mit hohem Chloridanteil bis zu reinen Silberchloridemulsionen verwendet. Es kann sich um überwiegend kompakte Kristalle handeln, die z.B. regulär kubisch oder oktaedrisch sind oder Übergangsformen aufweisen können. Vorzugsweise können aber auch plättchenförmige Kristalle vorliegen, deren durchschnittliches Verhältnis von Durchmesser zu Dicke bevorzugt wenigstens 5:1 ist, wobei der Durchmesser eines Kornes definiert ist als der Durchmesser eines Kreises mit einem Kreisinhalt entsprechend der projizierten Fläche des Kornes Die Schichten können aber auch tafelförmige Silberhalogenidkristalle aufweisen, bei denen das Verhältnis von Durchmesser zu Dicke wesentlich größer als 5:1 ist, z.B. 12:1 bis 30:1.

Die Silberhalogenidkörner können auch einen mehrfach geschichteten Kornaufbau aufweisen, im einfachsten Fall mit einem inneren und einem äußeren Kornbereich (core/shell), wobei die Halogenidzusammensetzung und/oder sonstige Modifizierungen, wie z.B. Dotierungen der einzelnen Kornbereiche unterschiedlich sind Die mittlere Korngröße der Emulsionen liegt vorzugsweise zwischen 0,2 µm und 2,0 µm, die Korngrößenverteilung kann sowohl homo- als auch heterodispers sein. Homodisperse Korngrößenverteilung bedeutet, daß 95 % der Körner nicht mehr als ± 30% von der mittleren Korngröße abweichen. Die Emulsionen können neben dem Silberhalogenid auch organische Silbersalze enthalten, z.B. Silberbenztriazolat oder Silberbehenat.

Es können zwei oder mehrere Arten von Silberhalogenidemulsionen, die getrennt hergestellt werden, als Mischung verwendet werden.

Als Bindemittel für die fotografischen Schichten wird vorzugsweise Gelatine verwendet. Diese kann jedoch ganz oder teilweise durch andere natürliche oder synthetische Bindemittel ersetzt werden.

Die Emulsionen können in der üblichen Weise chemisch und spektral sensibilisiert sein, und die Emulsionsschichten wie auch andere nicht-lichtempfindliche Schichten können in der üblichen Weise mit bekannten Härtungsmitteln gehärtet sein.

Üblicherweise enthalten farbfotografische Aufzeichnungsmaterialen mindestens je eine Silberhalogenidemulsionsschicht für die Aufzeichnung von Licht der drei Spektralbereiche Rot, Grün und Blau, Zu diesem Zweck sind die lichtempfindlichen Schichten in bekannter Weise durch geeignete Sensibilisierungsfarbstoffe spektral sensibilisiert, Blauempfindliche Silberhalogenidemulsionsschichten müssen nicht notwendigerweise einen Spektralsensibilisator enthalten, da für die Aufzeichnung von blauem Licht in vielen Fällen die Eigenempfindlichkeit des Silberhalogenids ausreicht.

Jede der genannten lichtempfindlichen Schichten kann aus einer einzigen Schicht bestehen oder in bekannter Weise, z.B. bei der sogenannten Doppelschichtanordnung, auch zwei oder mehr Silberhalogenidemulsionsteilschichten umfassen (DE-C-1 121 470). Üblicherweise sind rotempfindliche Silberhalogenidemulsionsschichten dem Schichtträger näher angeordnet als grünempfindliche Silberhalogenidemulsionsschichten und diese wiederum näher als blauempfindliche, wobei sich im allgemeinen zwischen grünempfindlichen Schichten und blauempfindlichen Schichten eine nicht lichtemfindliche gelbe Filterschicht befindet. Es sind aber auch andere Anordnungen denkbar. Zwischen Schichten unterschiedlicher Spektralempfindlichkeit ist in der Regel eine nicht lichtempfindliche Zwischenschicht angeordnet, die Mittel zur Unterbindung der Fehldiffusion von Entwickleroxidationsprodukten enthalten kann Falls mehrere Silberhalogenidemulsionsschichten gleicher Spektralempfindlichkeit vorhanden sind, können diese einander unmittelbar benachbart sein oder so angeordnet sein, daß sich zwischen ihnen eine lichtempfindliche Schicht mit anderer Spektralempfindlichkeit befindet (DE-A-1 958 709, DE-A-2 530 645, DE-A-2 622 922).

Farbfotografische Aufzeichnungsmaterialien zur Herstellung mehrfarbiger Bilder enthalten üblicherweise in räumlicher und spektraler Zuordnung zu den Silberhalogenidemulsionsschichten unterschiedlicher Spektralempfindlichkeit farbgebende Verbindungen, hier besonders Farbkuppler, zur Erzeugung der unterschiedlichen Teilfarbenbilder Cyan, Magenta und Gelb.

Unter räumlicher Zuordnung ist dabei zu verstehen, daß der Farbkuppler sich in einer solchen räumlichen Beziehung zu der Silberhalogenidemulsionsschicht befindet, daß eine Wechselwirkung zwischen ihnen möglich ist, die eine bildgemäße Übereinstimmung zwischen dem bei der Entwicklung gebildeten Silberbild und dem aus dem Farbkuppler erzeugten Farbbild zuläßt. Dies wird in der Regel dadurch erreicht, daß der Farbkuppler in der Silberhalogenidemulsionsschicht selbst enthalten ist oder in einer hierzu benachbarten gegebenenfalls nichtlichtempfindlichen Bindemittelschicht.

Unter spektraler Zuordnung ist zu verstehen, daß die Spektralempfindlichkeit jeder der lichtempfindlichen Silberhalogendemulsionsschichten und die Farbe des aus dem jeweils räumlich zugeordneten Farbkuppler erzeugten Teilfarbenbildes in einer bestimmten Beziehung zueinander stehen, wobei jeder der Spektralempfindlichkeiten (Rot, Grün, Blau) eine andere Farbe des betreffenden Teilfarbenbildes (z.B. Cyan, Magenta, Gelb) zugeordnet ist.

Jeder der unterschiedlich spektral sensibilisierten Silberhalogenidemulsionsschichten kann ein oder können auch mehrere Farbkuppler zugeordnet sein Wenn mehrere Silberhalogenidemulsionsschichten gleicher Spektralempfindlichkeit vorhanden sind, kann jede von ihnen einen Farbkuppler enthalten, wobei diese Farbkuppler nicht notwendigerweise identisch zu sein brauchen. Sie sollen lediglich bei der Farbentwicklung wenigstens annähernd die gleiche Farbe ergeben, normalerweise eine Farbe, die komplementär ist zu der Farbe des Lichtes, für das die betreffenden Silberhalogenidemulsionsschichten überwiegend empfindlich sind.

Rotempfindlichen Silberhalogenidemulsionsschichten ist folglich bei bevorzugten Ausführungsformen mindestens ein nichtdiffundierender Farbkuppler zur Erzeugung des blaugrünen Teilfarbenbildes zugeordnet, im vorliegenden Fall mindestens ein Kuppler der Formel I. Grünempfindlichen Silberhalogenidemulsionsschichten ist mindestens ein nichtdiffundierender Farbkuppler zur Erzeugung des purpurnen Teilfarbenbildes zugeordnet, wobei üblicherweise Farbkuppler vom Typ des 5-Pyrazolons, des Indazolons oder des Pyrazoloazols Verwendung finden. Blauempfindlichen Silberhalogenidemulsionsschichten schließlich ist mindestens ein nichtdiffundierender Farbkuppler zur Erzeugung des gelben Teilfarbenbildes zugeordnet, in der Regel ein Farbkuppler mit einer offenkettigen Ketomethylengruppierung. Farbkuppler dieser Art sind in großer Zahl bekannt und in einer Vielzahl von Patentschriften beschrieben. Beispielhaft sei hier auf die Veröffentlichungen "Farbkuppler" von W. PELZ in "Mitteilungen aus den Forschungslaboratorien der Agfa, Leverkusen/München", Band III, Seite 111 (1961) und von K. VENKATARAMAN in "The Chemistry of Synthetic Dyes", Vol. 4, 341 bis 387, Academic Press (1971), verwiesen.

Bei den Farbkupplern kann es sich sowohl um übliche 4-Äquivalentkuppler handeln als auch um 2-Äquivalentkuppler, bei denen zur Farberzeugung eine geringere Menge Silberhalogenid erforderlich ist. 2-Äquivalentkuppler leiten sich bekanntlich von den 4-Äquivalentkupplern dadurch ab, daß sie in der

Kupplungsstelle einen Substituenten enthalten, der bei der Kupplung abgespalten wird. Zu den 2-Äquivalentkupplern sind sowohl solche zu rechnen, die praktisch farblos sind, als auch solche, die eine intensive Eigenfarbe aufweisen, die bei der Farbkupplung verschwindet bzw. durch die Farbe des erzeugten Bildfarbstoffes ersetzt wird. Letztere Kuppler können ebenfalls zusätzlich in den lichtempfindlichen Silberhalogenidemulsionsschichten vorhanden sein und dort als Maskenkuppler zur Kompensierung der unerwünschten Nebendichten der Bildfarbstoffe dienen. Zu den 2-Äquivalenkupplern sind aber auch die bekannten Weißkuppler zu rechnen, die jedoch bei Reaktion mit Farbentwickleroxidationsprodukten keinen Farbstoff ergehen. Zu den 2-Äquivalentkupplern sind ferner die bekannten DIR-Kuppler zu rechnen, bei denen es sich um Kuppler handelt, die in der Kupplungsstelle einen abspaltbaren Rest enthalten, der bei Reaktion mit Farbentwickleroxidationsprodukten als diffundierender Entwicklungsinhibitor in Freiheit gesetzt wird. Auch andere fotografisch wirksame Verbindungen, z.B. Entwicklungsbeschleuniger oder Schleiermittel, können bei der Entwicklung aus solchen Kupplern freigesetzt werden.

Über die genannten Bestandteile hinaus kann das farbfotografische Aufzeichnungsmaterial der vorliegenden Erfindung weitere Zusätze enthalten, zum Beispiel Antioxidantien, farbstoffstabilisierende Mittel und Mittel zur Beeinflussung der mechanischen und elektrostatischen Eigenschaften. Um die nachteilige Einwirkung von UV-Licht auf die mit dem erfindungsgemäßen farbfotografischen Aufzeichnungsmaterial hergestellten Farbbilder zu vermindern oder zu vermeiden, ist es vorteilhaft, in einer oder mehreren der in dem Aufzeichnungsmaterial enthaltenen Schichten, vorzugsweise in einer der oberen Schichten, UV-absorbierende Verbindungen zu verwenden. Geeignete UV-Absorber sind beispielsweise in US-A-3 253 921, DE-C-2 036 719 und EP-A-0 057 160 beschrieben.

Für die erfindungsgemäßen Materialien können die üblichen Schichtträger verwendet werden, siehe Research Disclosure Nr. 17 643, Abschnitt XVII.

Als Schutzkolloid bzw. Bindemittel für die Schichten des Aufzeichnungsmaterials sind die üblichen hydrophilen filmbildenden Mittel geeignet, z.B. Proteine, insbesondere Gelatine. Begußhilfsmittel und Weichmacher können verwendet werden. Verwiesen wird auf die in der oben angegebenen Research Disclosure 17 643 in Abschnitt IX, XI und XII angegebenen Verbindungen.

Die Schichten des fotografischen Materials können in der üblichen Weise gehärtet sein, beispielsweise mit Härtern des Epoxidtyps, des heterocyclischen Ethylenimins und des Acryloyltyps. Weiterhin ist es auch möglich, die Schichten gemäß dem Verfahren der deutschen Offenlegungsschrift 2 218 009 zu härten, um farbfotografische Materialien zu erzielen, die für eine Hochtemperaturverarbeitung geeignet sind. Es ist ferner möglich, die fotografischen Schichten mit Härtern der Diazin-, Triazin- oder 1,2-Dihydrochinolin-Reihe zu härten oder mit Härtern vom Vinylsulfon-Typ. Weitere geeignete Härtungsmittel sind aus den deutschen Offenlegungsschriften 2 439 551, 2 225 230, 2 317 672 und aus der oben angegebenen Research Disclosure 17 643, Abschnitt XI bekannt.

Weitere geeignete Zusätze werden in der Research Disclosure 17 643 und in "Product Licensing Index" von Dezember 1971, Seiten 107-110, angegeben.

Zur Herstellung farbfotografischer Bilder wird das erfindungsgemäße farbfotografische Aufzeichnungsmaterial, mit einer Farbentwicklerverbindung entwickelt Als Farbentwicklerverbindung lassen sich sämtliche Entwicklerverbindungen verwenden, die die Fähigkeit besitzen in Form ihres Oxidationsproduktes mit Farbkupplern zu Azomethinfarbstoffen zu reagieren. Geeignete Farbentwicklerverbindungen sind aromatische mindestens eine primäre Aminogruppe enthaltende Verbindungen vom p-Phenylendiamintyp, beispielsweise N,N-Dialkyl-p-phenylendiamine, wie N,N-Diethyl-p-phenylendiamin, 1-(N-ethyl-N-methylsulfonamidoethyl)-3-methyl-p-phenylendiamin, 1-(N-ethyl-N-hydroxyethyl-3-methyl-p-phenylendiamin und 1-(N-ethyl-N-methoxyethyl)-3-methyl-p-phenylendiamin.

Weitere brauchbare Farbentwickler sind beispielsweise beschrieben in J. Amer. Chem. Soc. 73, 3100 (1951) und in G. Haist, Modern Photographic Processing, 1979, John Wiley and Sons, New York, Seiten 545 ff.

Nach der Farbentwicklung wird das Material üblicherweise gebleicht und fixiert. Bleichung und Fixierung können getrennt voneinander oder auch zusammen durchgeführt werden. Als Bleichmittel können die üblichen Verbindungen verwendet werden, z.B. $Fe^{3+}$-Salze und $Fe^{3+}$-Komplexsalze wie Ferricyanide, Dichromate, wasserlösliche Kobaltkomplexe usw. Besonders bevorzugt sind Eisen-III-Komplexe von Aminopolycarbonsäuren insbesondere z.B. Ethylendiamintetraessigsäure, N-Hydroxyethylethylendiamintriessigsäure, Alkyliminodicarbonsäuren und von entsprechenden Phosphonsäuren. Geeignet als Bleichmittel sind weiterhin Persulfate.

Beispiel 1

Vergleich des erfindungsgemäßen Kupplers Nr. 6 mit den Verbindungen VK 1 und VK 2

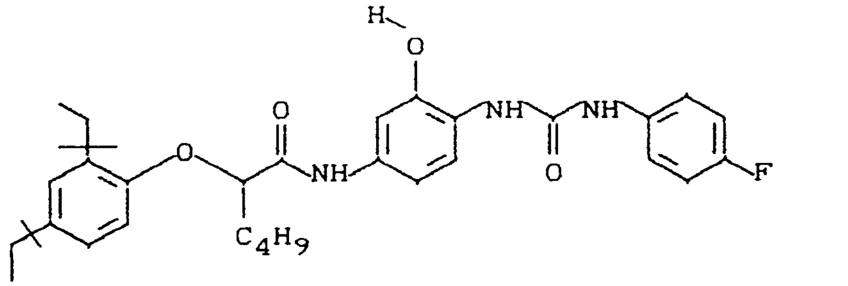

VK 1

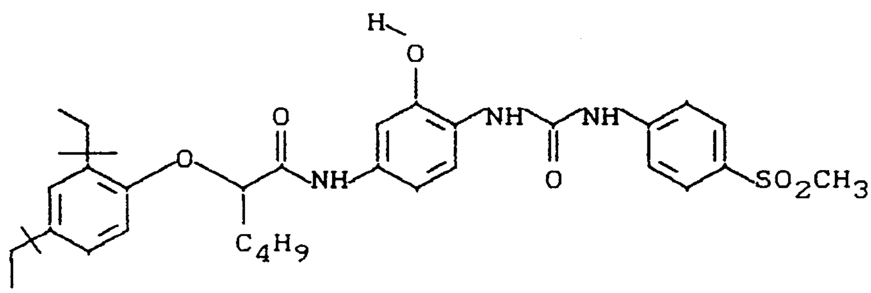

VK 2

Jeweils 8 mmol Kuppler werden im Verhältnis 1:3 in ca. 50 °C warmem Ethylacetat (EA) gelöst und mit Dibutylphthalat (DBP) versetzt, so daß ein Verhältnis von

Kuppler : DBP : EA : Manoxol = 1 : 1 : 3 : 0,1

resultiert Anschließend wird in 7,5 %iger Gelatinelösung dispergiert. Abhängig vom Molgewicht des Kupplers ergibt sich ein Verhältnis Kuppler : Gelatine von ca. 1 : 2. Das Emulgat wurde 6 Minuten bei 1000 U/m gerührt, wobei es sich auf ca. 50 °C erwärmte und wobei EA im Wasserstrahlvakuum (200-300 mbar) abgesaugt wurde. Die Qualität der frischen Kuppleremulgate wurde mit Hilfe eines Phasenkontrastmikroskopes bzw. eines Polarisationsmikroskopes folgendermaßen bewertet:

a) Teilchengröße 1 = sehr fein
   2 = fein
   3 = fein mit einigen größeren Teilchen
   4 = mittel
   5 = grob

b) Homogenität 1 = keine Kristalle erkennbar
   2 = vereinzelte Kristalle erkennbar
   3 = viele Kristalle erkennbar
   4 = stark auskristallisiert

Tabelle 1

| Qualität der Kuppleremulgate | | | | | | |
|---|---|---|---|---|---|---|
| | frisch | | nach 3 h 50 ° C | | nach 6 h 50 ° C | |
| | a | b | a | b | a | b |
| Kuppler Nr. 6 | 1 | 1 | 1 | 1 | 1 | 2 |
| VK 1 | 2 | 2 | 2 | 3 | 3 | 3 |
| VK 2 | 2 | 3 | 3 | 3 | 3 | 3 |

Beispiel 2

Die gemäß Beispiel 1 hergestellten Emulgate wurden mit einer Silberbromidiodidemulsion (0,7 mol-% Iodid) im Verhältnis 1 mol Kuppler : 5,2 mol $AgNO_3$ abgemischt, auf einen Schichtträger aus Celluloseacetat aufgetragen und mit einer Schutzschicht aus einer 3 %igen Gelatinelösung überschichtet, die als Härtungsmittel ein Cabamoylpyridiniumbetain (CAS Reg. No. 65411-60-1) enthielt. Nach dem Trocknen und Aufschneiden wurden die so hergestellten Proben hinter einem Stufenkeil belichtet und in einem Negativ-AP 79 Prozeß bei 38 ° C verarbeitet.

| Bad | Minuten |
|---|---|
| Farbentwickler CD 70 | 3,25 |
| Bleichbad | 6,50 |
| Wässerung | 3,0 |
| Fixierbad | 6,5 |
| Wässerung | 6,0 |

Folgende Bäder wurden verwendet:

Farbentwickler

8000 ml Wasser
17 g Hydroxyethandiphosphonsäure Na
12 g Ethylendiamintetraessigsäure (EDTA-Säure)
47 g 1-(N-Ethyl-N-hydroxyethyl)-3-methyl-p-phenylendiamin
25 g Hydroxylammoniumsulfat
39 g Natriumsulfit
15,5 g Natriumhydrogencarbonat
335 g Kaliumcarbonat
13,5 g Kaliumbromid
mit Wasser auf 10 l auffüllen; pH 10,0

Bleichbad

8000 ml Wasser
1390 g Ammoniumbromid
865 g EDTA $NH_4$-Fe
163 g EDTA-Säure
100 g Ammoniak
mit Wasser auf 10 l auffüllen und mit ca.
15 ml Eisessig auf pH 6,0 + /-0,1 einstellen

Fixierbad

8000 ml Wasser
1500 g Ammoniumthiosulfat
100 g Natriumsulfit
20 g Natriumhexamethaphosphat
mit Wasser auf 10 l auffüllen; pH 7,5

Neben den Kupplern aus Beispiel 1 wurden folgende Verbindungen in analoger Weise eingesetzt: VK 3; VK 4; VK 5 sowie die erfindungsgemäßen Kuppler Nr. 1;

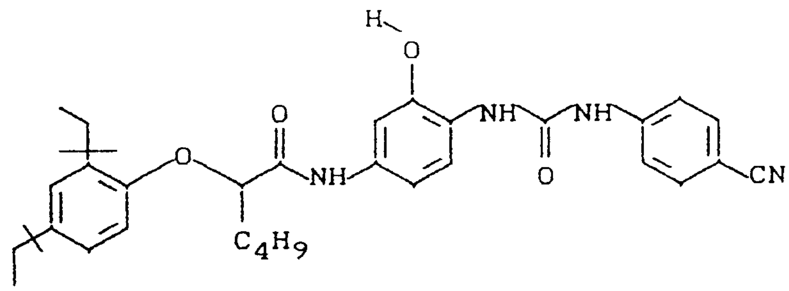

VK 3

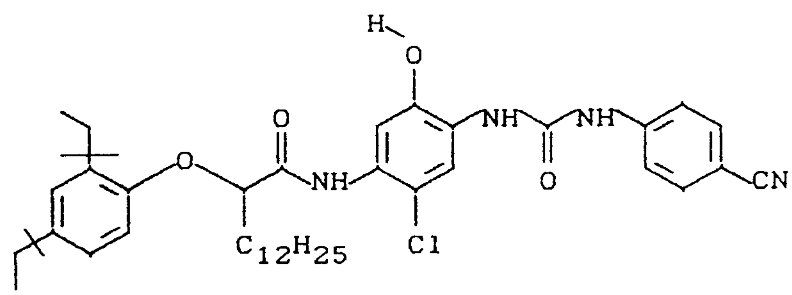

VK 4

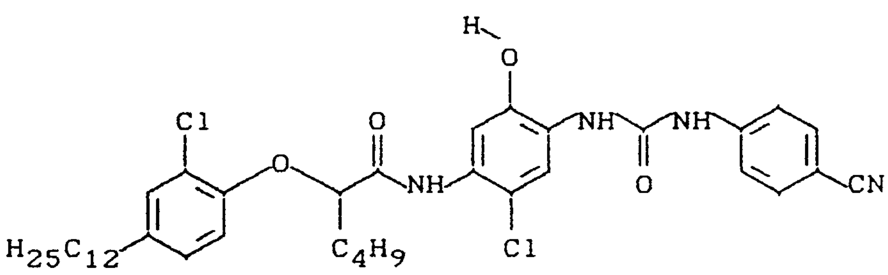

VK 5

Tabelle 2

| | E | ν | $D_{max}$ | FA | S | $\lambda_{max}$ | hbw |
|---|---|---|---|---|---|---|---|
| VK 1 | -0,3 | 2,5 | 2,6 | 1,77 | 0,11 | 679 | 90 |
| VK 2 | -0,6 | 2,0 | 2,4 | 1,80 | 0,14 | 688 | 93 |
| VK 3 | Typ | 3,0 | 2,5 | 1,79 | 0,09 | 692 | 95 |
| VK 4 | +0,3 | 2,9 | 3,0 | 1,90 | 0,19 | 692 | 95 |
| VK 5 | -0,6 | 2,3 | 2,4 | 1,81 | 0,12 | 684 | 88 |
| Kuppler Nr. 6 | = Typ | 3,5 | 3,6 | 2,33 | 0,09 | 684 | 78 |
| Kuppler Nr. 1 | +0,6 | 3,6 | 3,5 | 2,44 | 0,10 | 696 | 80 |
| Kuppler Nr. 3 | +0,3 | 3,7 | 3,6 | 2,40 | 0,10 | 692 | 86 |
| Kuppler Nr. 7 | +1,0 | 3,4 | 3,4 | 2,30 | 0,11 | 690 | 86 |

Aus der Tabelle 2 geht hervor, daß die Kuppler gemäß der Erfindung gegenüber den Vergleichskupplern bei vergleichbarem Schleier S eine tendenziell höhere Empfindlichkeit E aufweisen. Vor allen Dingen aber zeichnen sich die erfindungsgemäßen Verbindungen durch eine steile Gradation (ν), eine hohe maximale Dichte ($D_{max}$) sowie eine ausgezeichnete Farbausbeute (FA) aus. Als Farbausbeute (FA) wird der Quotient aus $D_{max}$ durch aufgetragenes Silber bezeichnet.

## Patentansprüche

1. Farbfotografisches Aufzeichnungsmaterial mit mindestens einer für den roten Spektralbereich sensibilisierten Silberhalogenidemulsionsschicht, der ein Cyankuppler vom Typ des 2-Phenylureidophenols zugeordnet ist, dadurch gekennzeichnet, daß der Cyankuppler der Formel I entspricht

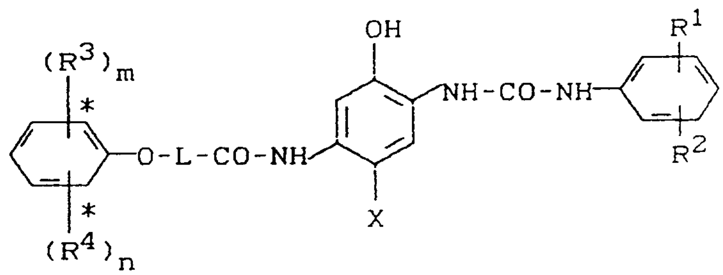

(I)

worin bedeuten

X H oder eine von Wasserstoff verschiedene, bei Farbkupplung freisetzbare Gruppe;
L ein zweibindiges aliphatisches Bindeglied, insbesondere Alkylen oder Alkyliden mit 1 - 17 C-Atomen;
$R^1$ einen elektronenanziehenden Substituenten;
$R^2$ H, Cl, Alkyl, Alkoxy, Alkylthio, Acylamino oder einen Rest wie $R^1$;
$R^3$ Cycloalkyl;
$R^4$ Halogen oder Alkoxy mit 1 bis 12 C-Atomen, wobei falls $R^4$ Alkoxy bedeutet, beide ortho-Positionen (*) substituiert sind;
m 1 oder 2;
n 1, 2 oder 3.

Europäisches Patentamt

# EUROPÄISCHER RECHERCHENBERICHT

Nummer der Anmeldung
EP 94 11 7334

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
|---|---|---|---|
| A | EP-A-0 434 028 (FUJI PHOTO FILM CO., LTD) 26. Juni 1991<br>* Seite 3, Zeile 1 - Seite 3, Zeile 50 *<br>* Seite 4, Zeile 52 - Seite 7, Zeile 29 *<br>* Verbindungen II-1 und II-15 *<br>--- | 1 | G03C7/34 |
| D,A | EP-A-0 028 099 (EASTMAN KODAK COMPANY) 6. Mai 1981<br>* Seite 1, Zeile 1 - Seite 2, Zeile 15 *<br>* Seite 5, Zeile 9 - Seite 7, Zeile 31 *<br>----- | 1 | |

RECHERCHIERTE SACHGEBIETE (Int.Cl.6)

G03C

Der vorliegende Recherchenbericht wurde für alle Patentansprüche erstellt

| Recherchenort | Abschlußdatum der Recherche | Prüfer |
|---|---|---|
| MÜNCHEN | 17. Februar 1995 | Markowski, V |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
.....................................................................
& : Mitglied der gleichen Patentfamilie, übereinstimmendes Dokument

EPO FORM 1503 03.82 (P04C03)